(19) Europäisches Patentamt
European Patent Office
Office européen des brevets

(11) EP 3 167 799 A1

(12) EUROPEAN PATENT APPLICATION
published in accordance with Art. 153(4) EPC

(43) Date of publication:
17.05.2017 Bulletin 2017/20

(51) Int Cl.:
A61B 5/0488 (2006.01)

(21) Application number: 15832528.2

(22) Date of filing: 27.02.2015

(86) International application number:
PCT/CN2015/073369

(87) International publication number:
WO 2016/023349 (18.02.2016 Gazette 2016/07)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 13.08.2014 CN 201410396422

(71) Applicant: Huawei Technologies Co., Ltd.
Longgang District
Shenzhen, Guangdong 518129 (CN)

(72) Inventors:
• DING, Qiang
Shenzhen
Guangdong 518129 (CN)
• GAO, Xiaorong
Beijing 100084 (CN)
• LIN, Ke
Beijing 100084 (CN)

(74) Representative: Maiwald Patentanwalts GmbH
Engineering
Elisenhof
Elisenstrasse 3
80335 München (DE)

(54) MOVEMENT RECOGNITION METHOD AND APPARATUS BASED ON SURFACE ELECTROMYOGRAPHIC SIGNAL

(57) An action recognition method based on a surface electromyography signal includes: obtaining surface electromyography signals of multiple channels (101 and 301); determining a valid surface electromyography signal according to the surface electromyography signals of the multiple channels (102 and 302); determining a frequency of the valid surface electromyography signal (103 and 303); and determining, according to the frequency of the valid surface electromyography signal, a body action corresponding to the surface electromyography signals of the multiple channels (104). A frequency of a surface electromyography signal is irrelevant to a feature such as signal strength; therefore, the method can significantly improve accuracy of action recognition based on a surface electromyography signal. Moreover, with a frequency being used as a recognition feature, a user does not need to conduct an action with a large range, which brings better user experience. In addition, the action recognition method based on a surface electromyography signal further includes: extracting an amplitude feature of the valid surface electromyography signal (304); and determining, according to the amplitude feature of the valid surface electromyography signal and the frequency of the valid surface electromyography signal, a body action corresponding to the surface electromyography signals of the multiple channels (305). The method can not only improve accuracy of recognizing a surface electromyography signal, but also can increase recognition types of body actions.

FIG. 1

**Description**

**TECHNICAL FIELD**

Embodiments of the present invention relate to action recognition technologies, and in particular, to an action recognition method and device based on a surface electromyography signal.

**BACKGROUND**

A surface electromyography signal (surface electromyography signal, SEMG) is a type of bioelectric signal related to a neuromuscular activity, and the surface electromyography signal can reflect information such as muscle contraction mode and contraction strength. Different body actions are corresponding to different surface electromyography signals, and a specific action corresponding to a surface electromyography signal may be determined by analyzing the surface electromyography signal. Therefore, a surface electromyography signal is widely applied to many fields such as clinical medicine, sport medicine, biomedicine, and rehabilitation engineering.

In the prior art, an amplitude feature of a surface electromyography signal is used as a recognition parameter to recognize a body action. According to the method, sliding window processing is performed on a collected surface electromyography signal, to obtain a window sequence of each sliding window; then, a window sequence amplitude of each window is calculated to obtain an amplitude feature, and the amplitude feature is compared with an amplitude feature of a surface electromyography signal corresponding to a body action that is obtained by means of pre-training, so as to determine a body action corresponding to the amplitude feature.

The prior-art method has the following problems: Because another user action may also generate a surface electromyography signal, when a user conducts another motion (running, typing, or the like), a generated surface electromyography interference signal may be overlapped with a target surface electromyography signal, and consequently, recognition accuracy is low; and in addition, a magnitude of a surface electromyography signal amplitude is proportional to a user body action amplitude, and to reach a relatively high signal-to-noise ratio, the user needs to conduct a relatively intense body action, which makes the user feel tired during a long-time operation.

**SUMMARY**

Embodiments of the present invention provide an action recognition method and device based on a surface electromyography signal, so that accuracy of action recognition based on a surface electromyography signal can be improved.

A first aspect of the present invention provides an action recognition method based on a surface electromyography signal, including:

  obtaining surface electromyography signals of multiple channels;
  determining a valid surface electromyography signal according to the surface electromyography signals of the multiple channels;
  determining a frequency of the valid surface electromyography signal; and
  determining, according to the frequency of the valid surface electromyography signal, a body action corresponding to the surface electromyography signals of the multiple channels.

With reference to the first aspect of the present invention, in a first possible implementation manner of the first aspect of the present invention, the determining a valid surface electromyography signal according to the surface electromyography signals of the multiple channels includes:

  overlapping the surface electromyography signals of the multiple channels, and dividing the overlapped surface electromyography signals by a quantity of the channels to obtain a single-channel surface electromyography signal;
  sliding, starting from a start time of the single-channel surface electromyography signal, the single-channel surface electromyography signal at each sliding moment to obtain a window corresponding to each sliding moment, determining a window sequence corresponding to each sliding moment, and calculating an average surface electromyography signal amplitude of the window sequence corresponding to each sliding moment, where there is one sliding interval between each sliding moment, the average surface electromyography signal amplitude of the window sequence is an average absolute value of amplitudes of surface electromyography signals within the window sequence, and the window sequence corresponding to the sliding moment includes a total of N consecutive windows: a window corresponding to the sliding moment and N-1 windows corresponding to N-1 sliding moments before the sliding moment, where N is a positive integer greater than or equal to 2; and

if an average surface electromyography signal amplitude of a window sequence corresponding to a sliding moment T in all sliding moments is not less than a preset amplitude, using a start time of the window sequence corresponding to the sliding moment T as a start time of the valid surface electromyography signal, adding a preset time to the start time of the valid surface electromyography signal to obtain an end time of the valid surface electromyography signal, and capturing and using a surface electromyography signal of the multiple channels that is located between the start time and the end time of the valid surface electromyography signal as the valid surface electromyography signal.

With reference to the first possible implementation manner of the first aspect of the present invention, in a second possible implementation manner of the first aspect of the present invention, the preset amplitude is an average absolute value of amplitudes of the surface electromyography signals obtained by overlapping the surface electromyography signals of the multiple channels.

With reference to the first aspect of the present invention and the first and second possible implementation manners of the first aspect of the present invention, in a third possible implementation manner of the first aspect of the present invention, the determining a frequency of the valid surface electromyography signal includes:

calculating a correlation coefficient between the valid surface electromyography signal and each of multiple sine-cosine matrices, where the sine-cosine matrix includes fundamental-frequency and multiplied-frequency sine functions and cosine functions, and all of the sine-cosine matrices have different fundamental frequencies;

determining whether a greatest correlation coefficient in the correlation coefficients between the valid surface electromyography signal and the multiple sine-cosine matrices is greater than a preset correlation coefficient; and

if the greatest correlation coefficient is greater than the preset correlation coefficient, using a fundamental frequency of a sine-cosine matrix corresponding to the greatest correlation coefficient as the frequency of the valid surface electromyography signal.

A second aspect of the present invention provides an action recognition method based on a surface electromyography signal, including:

obtaining surface electromyography signals of multiple channels;

determining a valid surface electromyography signal according to the surface electromyography signals of the multiple channels;

determining a frequency of the valid surface electromyography signal;

extracting an amplitude feature of the valid surface electromyography signal; and

determining, according to the amplitude feature of the valid surface electromyography signal and the frequency of the valid surface electromyography signal, a body action corresponding to the surface electromyography signals of the multiple channels.

With reference to the second aspect of the present invention, in a first possible implementation manner of the second aspect of the present invention, the determining a valid surface electromyography signal according to the surface electromyography signals of the multiple channels includes:

overlapping the surface electromyography signals of the multiple channels, and dividing the overlapped surface electromyography signals by a quantity of the channels to obtain a single-channel surface electromyography signal; sliding, starting from a start time of the single-channel surface electromyography signal, the single-channel surface electromyography signal at each sliding moment to obtain a window corresponding to each sliding moment, determining a window sequence corresponding to each sliding moment, and calculating an average surface electromyography signal amplitude of the window sequence corresponding to each sliding moment, where there is one sliding interval between each sliding moment, the average surface electromyography signal amplitude of the window sequence is an average absolute value of amplitudes of surface electromyography signals within the window sequence, and the window sequence corresponding to the sliding moment includes a total of N consecutive windows: a window corresponding to the sliding moment and N-1 windows corresponding to N-1 sliding moments before the sliding moment, where N is a positive integer greater than or equal to 2; and

when an average surface electromyography signal amplitude of a window sequence corresponding to a sliding moment T in all sliding moments is not less than a preset amplitude, using a start time of the window sequence corresponding to the sliding moment T as a start time of the valid surface electromyography signal, adding a preset time to the start time of the valid surface electromyography signal to obtain an end time of the valid surface electromyography signal, and capturing and using a surface electromyography signal of the multiple channels that is located between the start time and the end time of the valid surface electromyography signal as the valid surface electromyography signal.

With reference to the first possible implementation manner of the second aspect of the present invention, in a second possible implementation manner of the second aspect of the present invention, the preset amplitude is an average absolute value of amplitudes of the surface electromyography signals obtained by overlapping the surface electromyography signals of the multiple channels.

With reference to the second aspect of the present invention and the first and second possible implementation manners of the second aspect of the present invention, in a third possible implementation manner of the second aspect of the present invention, the determining a frequency of the valid surface electromyography signal includes:

calculating a correlation coefficient between the valid surface electromyography signal and each of multiple sine-cosine matrices, where the sine-cosine matrix includes fundamental-frequency and multiplied-frequency sine functions and cosine functions, and all of the sine-cosine matrices have different fundamental frequencies; determining whether a greatest correlation coefficient in the correlation coefficients between the valid surface electromyography signal and the multiple sine-cosine matrices is greater than a preset correlation coefficient; and if the greatest correlation coefficient is greater than the preset correlation coefficient, using a fundamental frequency of a sine-cosine matrix corresponding to the greatest correlation coefficient as the frequency of the valid surface electromyography signal.

With reference to the third possible implementation manner of the second aspect of the present invention, in a fourth possible implementation manner of the second aspect of the present invention, the extracting an amplitude feature of the valid surface electromyography signal includes:

separately performing sliding window processing on surface electromyography signals of each channel of the valid surface electromyography signal; and calculating an average amplitude of each sliding window of the surface electromyography signals of each channel of the valid surface electromyography signal, where the average amplitude of each sliding window is an average absolute value of amplitudes of surface electromyography signals within each sliding window, and using the average amplitude of each sliding window of the valid surface electromyography signal as the amplitude feature of the valid surface electromyography signal.

With reference to the second aspect of the present invention and the first and second possible implementation manners of the second aspect of the present invention, in a fifth possible implementation manner of the second aspect of the present invention, the determining, according to the amplitude feature of the valid surface electromyography signal and the frequency of the valid surface electromyography signal, a body action corresponding to the surface electromyography signals of the multiple channels includes:

determining, according to the frequency of the valid surface electromyography signal, multiple alternative body actions corresponding to the surface electromyography signals of the multiple channels; and performing matching between the amplitude feature of the valid surface electromyography signal and an amplitude feature of the multiple alternative body actions that is obtained by means of pre-training, to obtain a body action matched with the amplitude feature of the valid surface electromyography signal, and using the body action matched with the amplitude feature of the valid surface electromyography signal as the body action corresponding to the surface electromyography signals of the multiple channels.

A third aspect of the present invention provides an action recognition device based on a surface electromyography signal, including:

an obtaining module, configured to obtain surface electromyography signals of multiple channels; a first determining module, configured to determine a valid surface electromyography signal according to the surface electromyography signals of the multiple channels; a second determining module, configured to determine a frequency of the valid surface electromyography signal; and a recognition module, configured to determine, according to the frequency of the valid surface electromyography signal, a body action corresponding to the surface electromyography signals of the multiple channels.

With reference to the third aspect of the present invention, in a first possible implementation manner of the third aspect of the present invention, the first determining module is specifically configured to:

overlap the surface electromyography signals of the multiple channels, and divide the overlapped surface electromyography signals by a quantity of the channels to obtain a single-channel surface electromyography signal; slide, starting from a start time of the single-channel surface electromyography signal, the single-channel surface electromyography signal at each sliding moment to obtain a window corresponding to each sliding moment, determine a window sequence corresponding to each sliding moment, and calculate an average surface electromyography signal amplitude of the window sequence corresponding to each sliding moment, where there is one sliding interval between each sliding moment, the average surface electromyography signal amplitude of the window sequence is an average absolute value of amplitudes of surface electromyography signals within the window sequence, and the window sequence corresponding to the sliding moment includes a total of N consecutive windows: a window corresponding to the sliding moment and N-1 windows corresponding to N-1 sliding moments before the sliding moment, where N is a positive integer greater than or equal to 2; and

if an average surface electromyography signal amplitude of a window sequence corresponding to a sliding moment T in all sliding moments is not less than a preset amplitude, use a start time of the window sequence corresponding to the sliding moment T as a start time of the valid surface electromyography signal, add a preset time to the start time of the valid surface electromyography signal to obtain an end time of the valid surface electromyography signal, and capture and use a surface electromyography signal of the multiple channels that is located between the start time and the end time of the valid surface electromyography signal as the valid surface electromyography signal.

With reference to the first possible implementation manner of the third aspect of the present invention, in a second possible implementation manner of the third aspect of the present invention, the preset amplitude is an average absolute value of amplitudes of the surface electromyography signals obtained by overlapping the surface electromyography signals of the multiple channels.

With reference to the third aspect of the present invention and the first and second possible implementation manners of the third aspect of the present invention, in a third possible implementation manner of the third aspect of the present invention, the second determining module is specifically configured to:

calculate a correlation coefficient between the valid surface electromyography signal and each of multiple sine-cosine matrices, where the sine-cosine matrix includes fundamental-frequency and multiplied-frequency sine functions and cosine functions, and all of the sine-cosine matrices have different fundamental frequencies; determine whether a greatest correlation coefficient in the correlation coefficients between the valid surface electromyography signal and the multiple sine-cosine matrices is greater than a preset correlation coefficient; and if the greatest correlation coefficient is greater than the preset correlation coefficient, use a fundamental frequency of a sine-cosine matrix corresponding to the greatest correlation coefficient as the frequency of the valid surface electromyography signal.

A fourth aspect of the present invention provides an action recognition device based on a surface electromyography signal, including:

an obtaining module, configured to obtain surface electromyography signals of multiple channels; a first determining module, configured to determine a valid surface electromyography signal according to the surface electromyography signals of the multiple channels; a second determining module, configured to determine a frequency of the valid surface electromyography signal; an extraction module, configured to extract an amplitude feature of the valid surface electromyography signal; and a recognition module, configured to determine, according to the amplitude feature of the valid surface electromyography signal and the frequency of the valid surface electromyography signal, a body action corresponding to the surface electromyography signals of the multiple channels.

With reference to the fourth aspect of the present invention, in a first possible implementation manner of the fourth aspect of the present invention, the first determining module is specifically configured to:

overlap the surface electromyography signals of the multiple channels, and divide the overlapped surface electromyography signals by a quantity of the channels to obtain a single-channel surface electromyography signal; slide, starting from a start time of the single-channel surface electromyography signal, the single-channel surface electromyography signal at each sliding moment to obtain a window corresponding to each sliding moment, determine a window sequence corresponding to each sliding moment, and calculate an average surface electromyography signal amplitude of the window sequence corresponding to each sliding moment, where there is one sliding interval between each sliding moment, the average surface electromyography signal amplitude of the window sequence is an average absolute value of amplitudes of surface electromyography signals within the window sequence, and the window sequence corresponding to the sliding moment includes a total of N consecutive windows: a window corresponding to the sliding moment and N-1 windows corresponding to N-1 sliding moments before the sliding moment, where N is a positive integer greater than or equal to 2; and

when an average surface electromyography signal amplitude of a window sequence corresponding to a sliding moment T in all sliding moments is not less than a preset amplitude, use a start time of the window sequence corresponding to the sliding moment T as a start time of the valid surface electromyography signal, adding a preset time to the start time of the valid surface electromyography signal to obtain an end time of the valid surface electromyography signal, and capture and use a surface electromyography signal of the multiple channels that is located between the start time and the end time of the valid surface electromyography signal as the valid surface electromyography signal.

With reference to the first possible implementation manner of the fourth aspect of the present invention, in a second possible implementation manner of the fourth aspect of the present invention, the preset amplitude is an average absolute value of amplitudes of the surface electromyography signals obtained by overlapping the surface electromyography signals of the multiple channels.

With reference to the fourth aspect of the present invention and the first and second possible implementation manners of the fourth aspect of the present invention, in a third possible implementation manner of the fourth aspect of the present invention, the second determining module is specifically configured to:

calculate a correlation coefficient between the valid surface electromyography signal and each of multiple sine-cosine matrices, where the sine-cosine matrix includes fundamental-frequency and multiplied-frequency sine functions and cosine functions, and all of the sine-cosine matrices have different fundamental frequencies;

determine whether a greatest correlation coefficient in the correlation coefficients between the valid surface electromyography signal and the multiple sine-cosine matrices is greater than a preset correlation coefficient; and

if the greatest correlation coefficient is greater than the preset correlation coefficient, use a fundamental frequency of a sine-cosine matrix corresponding to the greatest correlation coefficient as the frequency of the valid surface electromyography signal.

With reference to the third possible implementation manner of the fourth aspect of the present invention, in a fourth possible implementation manner of the fourth aspect of the present invention, the extraction module is specifically configured to:

separately perform sliding window processing on surface electromyography signals of each channel of the valid surface electromyography signal; and

calculate an average amplitude of each sliding window of the surface electromyography signals of each channel of the valid surface electromyography signal, where the average amplitude of each sliding window is an average absolute value of amplitudes of surface electromyography signals within each sliding window, and use the average amplitude of each sliding window of the valid surface electromyography signal as the amplitude feature of the valid surface electromyography signal.

With reference to the fourth aspect of the present invention and the first and second possible implementation manners of the fourth aspect of the present invention, in a fifth possible implementation manner of the fourth aspect of the present invention, the recognition module is specifically configured to:

determine, according to the frequency of the valid surface electromyography signal, multiple alternative body actions corresponding to the surface electromyography signals of the multiple channels; and

perform matching between the amplitude feature of the valid surface electromyography signal and an amplitude feature of the multiple alternative body actions that is obtained by means of pre-training, to obtain a body action matched with the amplitude feature of the valid surface electromyography signal, and use the body action matched with the amplitude feature of the valid surface electromyography signal as the body action corresponding to the surface electromyography signals of the multiple channels.

A fifth aspect of the present invention provides an action recognition device based on a surface electromyography signal, including:

a processor, a memory, and a system bus, where by using the system bus, the processor and the memory are connected and implement mutual communication;

the memory is configured to store a computer execution instruction; and

the processor is configured to run the computer execution instruction and enable the action recognition device to execute the method according to the first aspect of the present invention and the first to third implementation manners of the first aspect of the present invention.

A sixth aspect of the present invention provides an action recognition device based on a surface electromyography signal, including:

a processor, a memory, and a system bus, where by using the system bus, the processor and the memory are connected and implement mutual communication;
the memory is configured to store a computer execution instruction; and
the processor is configured to run the computer execution instruction and enable the action recognition device to execute the method according to the second aspect of the present invention and the first to fifth implementation manners of the second aspect of the present invention.

According to the action recognition method and device based on a surface electromyography signal provided in the embodiments of the present invention, surface electromyography signals of multiple channels are obtained, and a valid surface electromyography signal is determined according to the surface electromyography signals of the multiple channels; then, a frequency of the valid surface electromyography signal is determined, and a body action corresponding to the surface electromyography signals of the multiple channels is determined according to the frequency of the valid surface electromyography signal. A frequency of a surface electromyography signal is irrelevant to signal strength or other features; therefore, the method in the embodiments can significantly improve accuracy of action recognition based on a surface electromyography signal. Moreover, with a frequency being used as a recognition feature, a user does not need to conduct an action with a large range, which brings better user experience. In addition, according to the method in the embodiments of the present invention, a body action corresponding to a surface electromyography signal may be further recognized with reference to a frequency and amplitude feature of the surface electromyography signal, which can not only improve accuracy of recognizing a surface electromyography signal, but also can increase recognition types of body actions.

**BRIEF DESCRIPTION OF DRAWINGS**

To describe the technical solutions in the embodiments of the present invention or in the prior art more clearly, the following briefly describes the accompanying drawings required for describing the embodiments or the prior art. Apparently, the accompanying drawings in the following description show some embodiments of the present invention, and a person of ordinary skill in the art may still derive other drawings from these accompanying drawings without creative efforts.

FIG. 1 is a flowchart of an action recognition method based on a surface electromyography signal according to Embodiment 1 of the present invention;
FIG. 2 is a flowchart of a method for determining a frequency of a valid surface electromyography signal according to Embodiment 2 of the present invention;
FIG. 3 is a flowchart of an action recognition method based on a surface electromyography signal according to Embodiment 3 of the present invention;
FIG. 4 is an overall block diagram of an action recognition method based on a surface electromyography signal according to Embodiment 4 of the present invention;
FIG. 5 is a schematic structural diagram of an action recognition device based on a surface electromyography signal according to Embodiment 5 of the present invention;
FIG. 6 is a schematic structural diagram of an action recognition device based on a surface electromyography signal according to Embodiment 6 of the present invention;
FIG. 7 is a schematic structural diagram of an action recognition device based on a surface electromyography signal according to Embodiment 7 of the present invention; and
FIG. 8 is a schematic structural diagram of an action recognition device based on a surface electromyography signal according to Embodiment 8 of the present invention.

**DESCRIPTION OF EMBODIMENTS**

To make the objectives, technical solutions, and advantages of the embodiments of the present invention clearer, the following clearly and completely describes the technical solutions in the embodiments of the present invention with reference to the accompanying drawings in the embodiments of the present invention. Apparently, the described embodiments are some but not all of the embodiments of the present invention. All other embodiments obtained by a person of ordinary skill in the art based on the embodiments of the present invention without creative efforts shall fall within the protection scope of the present invention.

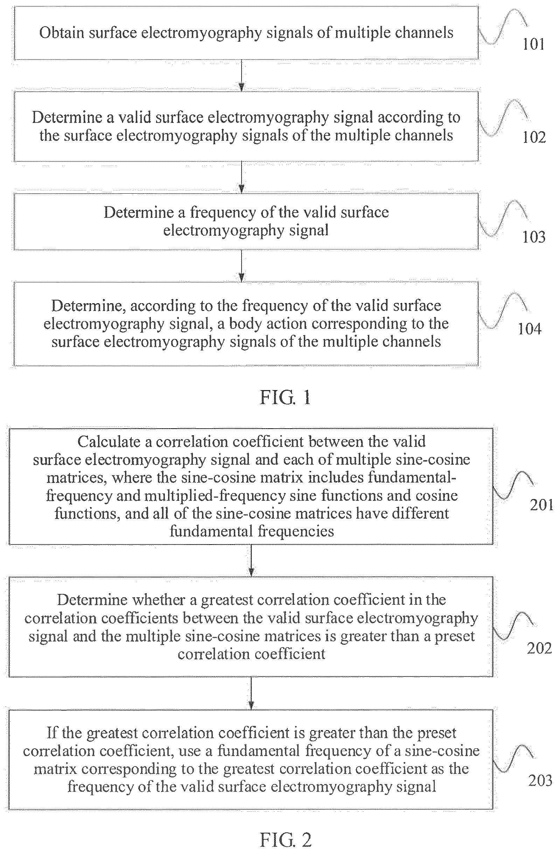

FIG. 1 is a flowchart of an action recognition method based on a surface electromyography signal according to Embodiment 1 of the present invention, and the method in this embodiment may be execute by an action recognition device based on a surface electromyography signal. The action recognition device based on a surface electromyography signal may be specifically a smartphone, a tablet, and another terminal device. As shown in FIG. 1, the method in this embodiment may include the following steps.

Step 101. Obtain surface electromyography signals of multiple channels.

The obtaining, by the action recognition device based on a surface electromyography signal, surface electromyography signals of multiple channels is specifically: receiving the surface electromyography signals, of the multiple channels, sent by a collection apparatus, namely, placing the collection apparatus on a muscle group surface whose signal is to be collected, to collect the surface electromyography signals. In the method of this embodiment, a corresponding body action is determined according to a frequency of a surface electromyography signal; therefore, a user is required to repeatedly conduct a same body action at a specific frequency when the surface electromyography signals are collected. The body action may be not only a rhythmic gesture of a human forearm, but also a rhythmic action of a leg, or even a rhythmic action of a neck, belly or another body part, and the rhythmic action herein is an action repeated at a frequency.

The collection apparatus includes multiple sensing nodes, data collected by one sensing node is used as a surface electromyography signal of one channel, and the collection apparatus may be built in a smart watch or band or another wearable device. When the user conducts a body action, multiple muscle groups may be driven to act; therefore, it is necessary to collect surface electromyography signals of the multiple muscle groups, and the collected signals of the multiple channels comprehensively reflect the user body action.

Step 102. Determine a valid surface electromyography signal according to the surface electromyography signals of the multiple channels.

Because a surface electromyography signal has a small amplitude and a low signal-to-noise ratio, the obtained surface electromyography signals of the multiple channels need to be preprocessed before the valid surface electromyography signal is determined, where the preprocessing the surface electromyography signals is specifically: performing signal amplification, power frequency filtering, high-pass filtering, and other processing on the surface electromyography signals.

There may be an interference signal in surface electromyography signals collected by the collection apparatus in the most beginning. For example, the user does not conduct a corresponding body action in time when the collection apparatus starts working; in this case, although the user does not conduct a body action, the collection apparatus can still collect a weak surface electromyography signal. The weak surface electromyography signal is an interference signal. Therefore, it is necessary to remove a possible interference signal to obtain the valid surface electromyography signal.

Specifically, the valid surface electromyography signal may be determined according to the following method.

Step 1: Overlap the preprocessed surface electromyography signals of the multiple channels, and divide the overlapped surface electromyography signals by a quantity of the channels to obtain a single-channel surface electromyography signal.
Step 2: Slide, starting from a start time of the single-channel surface electromyography signal, the single-channel surface electromyography signal at each sliding moment to obtain a window corresponding to each sliding moment, determine a window sequence corresponding to each sliding moment, and calculate an average surface electromyography signal amplitude of the window sequence corresponding to each sliding moment, where there is one sliding interval between each sliding moment, the average surface electromyography signal amplitude of the window sequence is an average absolute value of amplitudes of surface electromyography signals within the window sequence, and the window sequence corresponding to the sliding moment includes a total of N consecutive windows: a window corresponding to the sliding moment and N-1 windows corresponding to N-1 sliding moments before the sliding moment, where N is a positive integer greater than or equal to 2.

The start time of the single-channel surface electromyography signal refers to a time when a channel signal starts to exist, the sliding the single-channel surface electromyography signal at each sliding moment is moving the single-channel surface electromyography signal backwards by one window in terms of time at each sliding moment, and a time difference between two adjacent sliding moments is a sliding interval. For example, a window width is a time period t, a sliding interval is t/2, where t=1 ms (millisecond), each window sequence includes four windows, there are a total of 50 windows, and window serial numbers are respectively 1, 2, 3...50. From the start time of the single-channel surface electromyography signal, a window corresponding to a first sliding moment is window No.4, a window sequence corresponding to the first sliding moment is 0-4 ms, and there are a total of four windows. At a second sliding moment, a single-channel surface electromyography signal is slid backwards by 0.5 ms, to obtain a second window sequence, the second window sequence is 0.5-4.5 ms, and by analogy, the window sequence corresponding to each sliding moment is obtained. To calculate an average surface electromyography signal amplitude of a window sequence corresponding to the first sliding moment, absolute values of amplitudes of single-channel surface electromyography signals within the window sequence are first obtained, then the absolute values of the single-channel surface electromyography signals within the window sequence are added to obtain an average value, thereby obtaining the average surface electromyography signal amplitude of the window sequence.

Step 3: If an average surface electromyography signal amplitude of a window sequence corresponding to a sliding moment T in all sliding moments is not less than a preset amplitude, use a start time of the window sequence corresponding to the sliding moment T as a start time of the valid surface electromyography signal, add a preset time to the start time of the valid surface electromyography signal to obtain an end time of the valid surface electromyography signal, and capture and use a surface electromyography signal of the multiple channels that is located between the start time and the end time of the valid surface electromyography signal as the valid surface electromyography signal.

When the average surface electromyography signal amplitude of the window sequence corresponding to each sliding moment is obtained by calculation, it is determined whether the average surface electromyography signal amplitude of the window sequence corresponding to the sliding moment is less than the preset amplitude. If the average surface electromyography signal amplitude of the window sequence corresponding to the sliding moment T is not less than the preset amplitude, that is, the average surface electromyography signal amplitude of the window sequence corresponding to the sliding moment T is greater than or equal to the preset amplitude, the start time of the window sequence corresponding to the sliding moment T is used as the start time of the valid surface electromyography signal, and the preset time is added to the start time of the valid surface electromyography signal to obtain the end time of the valid surface electromyography signal. After the start time and the end time of the valid surface electromyography signal is obtained, a surface electromyography signal of the multiple channels that is located between the start time and the end time of the valid surface electromyography signal are captured and used as the valid surface electromyography signal. The preset amplitude may be an average absolute value of amplitudes of the surface electromyography signals obtained by overlapping the surface electromyography signals of the multiple channels.

Step 103. Determine a frequency of the valid surface electromyography signal.

There are multiple methods for determining the frequency of the valid surface electromyography signal, and the following enumerates several commonly used methods. In a first method, a correlation coefficient between the valid surface electromyography signal and each of multiple sine-cosine matrices is calculated. Assuming that possible body action frequencies are f1, ..., fn, sine-cosine matrices with f1, ..., fn as fundamental frequencies are selected. Canonical correlation analysis (Canonical Correlation Analysis, CCA for short) operation is separately performed on the valid surface electromyography signal and each sine-cosine matrix, to obtain a greatest correlation coefficient between the valid surface electromyography signal and the sine-cosine matrices. If the greatest correlation coefficient is greater than a preset correlation coefficient, a fundamental frequency of a sine-cosine matrix corresponding to the greatest correlation coefficient is used as the frequency of the valid surface electromyography signal. In a second method, a Fast Fourier Transform (Fast Fourier Transform, FFT for short) is performed on the valid surface electromyography signal, and frequency distribution of the valid surface electromyography signal is determined according to a transform result, thereby obtaining the frequency of the valid surface electromyography signal. In a third method, the frequency of the surface electromyography signal is calculated according to two zero-crossing time intervals of the valid surface electromyography signal.

Step 104. Determine, according to the frequency of the valid surface electromyography signal, a body action corresponding to the surface electromyography signals of the multiple channels.

A correspondence between a frequency of a surface electromyography signal and a body action needs to be pre-established. For example, if a body action corresponding to a 1 HZ frequency is "clenching a fist", a body action corresponding to a 2 HZ frequency is "bending an elbow", and a body action corresponding to a 3 HZ frequency is an "OK gesture", when the user conducts the action of clenching a fist at the 1 HZ frequency, it is determined, according to the obtained frequency of the valid surface electromyography signal, that the body action of the user is clenching a fist.

In this embodiment of the present invention, surface electromyography signals of multiple channels are obtained, and a valid surface electromyography signal is determined according to the surface electromyography signals of multiple channels, then, a frequency of the valid surface electromyography signal is determined, and finally, a body action corresponding to the surface electromyography signals of multiple channels is determined according to the frequency of the surface electromyography signal. A frequency of a surface electromyography signal is irrelevant to signal strength or other features; therefore, the method in the embodiments can significantly improve accuracy of action recognition based on a surface electromyography signal. Moreover, with a frequency being used as a recognition feature, a user does not need to conduct an action with a large range, which brings better user experience.

In addition, with good anti-interference performance, the method in this embodiment can effectively resist electromyography noise interference generated by an irrelevant action, and the method can be applied when the user is in a non-stationary condition, such as running, driving, or doing housework. The method also provides high stability and is not affected by factors, such as a change of skin moisture, a change of an electrode contact status, and a muscle fatigue degree during a use process. The method provides the user with use convenience, user training data does not need to be pre-collected, and retraining is not required each time before use.

Embodiment 2 of the present invention elaborates Step 103 in Embodiment 1 of the present invention. FIG. 2 is a flowchart of a method for determining a frequency of a valid surface electromyography signal according to Embodiment 2 of the present invention. As shown in FIG. 2, the method in this embodiment includes the following steps.

Step 201. Calculate a correlation coefficient between the valid surface electromyography signal and each of multiple sine-cosine matrices, where the sine-cosine matrix includes fundamental-frequency and multiplied-frequency sine functions and cosine functions, and all of the sine-cosine matrices have different fundamental frequencies.

Assuming that possible body action frequencies are f1, ..., fn, sine-cosine matrices with f1, ..., fn as fundamental frequencies are selected for contrast. CCA operation is separately performed on the valid surface electromyography signal and the sine-cosine matrices, to obtain the correlation coefficients between the valid surface electromyography signal and each sine-cosine matrix.

Valid surface electromyography signals of n channels are recorded as $x = (x_1, x_2, x_3, ..., x_n)^T$, where $x_1, x_2, x_3, ..., x_n$ respectively represent a signal of each channel, and a sine-cosine matrix is $y = [\cos(2\pi f_1 t), \sin(2\pi f_1 t), \cos(4\pi f_1 t), \sin(4\pi f_1 t)]^T$, where f1 is a fundamental frequency. In this case, the CCA may be defined as the following problem: separately finding vectors $w_x$ and $w_y$, so that a correlation value between projections $X = x^T w_x$ and $Y = y^T w_y$ that are of x and y on the vectors $w_x$ and $w_y$ is the greatest, namely, p in the following formula is the greatest.

$$\rho = \frac{E[XY]}{\sqrt{E[X^2]E[Y^2]}} = \frac{E[w_x^T xy^T w_y]}{\sqrt{E[w_x^T xx^T w_x]E[w_y^T yy^T w_y]}} = \frac{w_x^T E[xy^T]w_y}{\sqrt{w_x^T E[xx^T]w_x w_y^T E[yy^T]w_y}} = \frac{w_y^T C_{xy}w_y}{\sqrt{w_x^T C_{xx}w_x w_y^T C_{yy}w_y}}$$

$C_{xy}$ indicates a cross correlation matrix of x and y, $C_{xx}$ indicates an autocorrelation matrix of x, and $C_{yy}$ indicates an autocorrelation matrix of y. A method for finding the vectors $w_x$ and $w_y$ is as follows:

Step 1. Construct the following Lagrangian by using the Lagrange algorithm:

$$L = w_x^T C_{xy}w_y - \frac{\lambda x}{2}(w_x^T C_{xx}w_x - 1) - \frac{\lambda y}{2}(w_y^T C_{yy}w_y - 1).$$

Step 2. Respectively seek for partial derivatives of L with respect to $w_x$ and $w_y$, to obtain:

$$C_{xy}w_y - \lambda_x C_{xx}w_x = 0$$

$$C_{yx}w_y - \lambda_y C_{yy}w_y = 0$$

In this way, formula 1 and formula 2 are obtained.

$$w_y = \frac{C_{yy}^{-1}C_{yx}w_x}{\lambda} \qquad (1)$$

$$\lambda = \lambda_x = \lambda_y, \quad C_{xx}^{-1}C_{xy}C_{yy}^{-1}C_{yx}w_x = \lambda^2 w_x \qquad (2)$$

$w_x$ can be obtained according to formula 2, and $w_y$ can be obtained according to formula 1. It can be learned from formula 2 that a process of obtaining $w_x$ according to formula 2 is converted into an Eigenvalue decomposition

problem, and $w_x$ and $w_y$ are put into the definition of p to obtain a correlation coefficient.

Step 202. Determine whether a greatest correlation coefficient in the correlation coefficients between the valid surface electromyography signal and the multiple sine-cosine matrices is greater than a preset correlation coefficient.

After correlation coefficients between the valid surface electromyography signal and all sine-cosine matrices are calculated, the greatest correlation coefficient is found out, to determine whether the greatest correlation coefficient is greater than the preset correlation coefficient.

Step 203. If the greatest correlation coefficient is greater than the preset correlation coefficient, use a fundamental frequency of a sine-cosine matrix corresponding to the greatest correlation coefficient as the frequency of the valid surface electromyography signal.

If the greatest correlation coefficient is greater than the preset correlation coefficient, it indicates that the valid surface electromyography signal has the frequency of the sine-cosine matrix corresponding to the greatest correlation coefficient. The fundamental frequency of the sine-cosine matrix corresponding to the greatest correlation coefficient is used as the frequency of the valid surface electromyography signal.

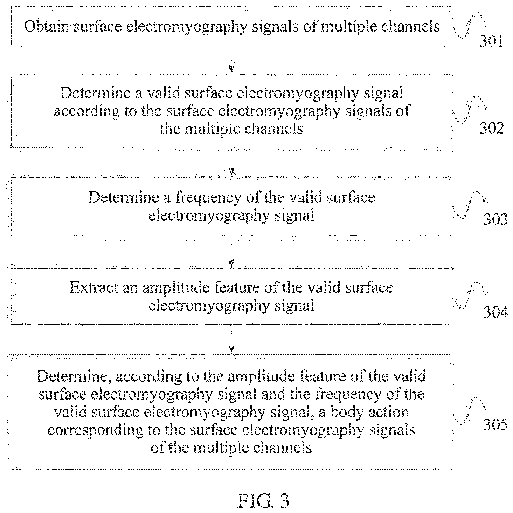

FIG. 3 is a flowchart of an action recognition method based on a surface electromyography signal according to Embodiment 3 of the present invention. A difference between the method in this embodiment and the method in Embodiment 1 is: in this embodiment, a body action corresponding to a surface electromyography signal is determined with reference to a frequency and an amplitude feature of the surface electromyography signal.

Step 301. Obtain surface electromyography signals of multiple channels.

Step 302. Determine a valid surface electromyography signal according to the surface electromyography signals of the multiple channels.

The determining a valid surface electromyography signal according to the surface electromyography signals of the multiple channels is specifically: overlapping the surface electromyography signals of the multiple channels, and dividing the overlapped surface electromyography signals by a quantity of the channels to obtain a single-channel surface electromyography signal; sliding, starting from a start time of the single-channel surface electromyography signal, the single-channel surface electromyography signal at each sliding moment to obtain a window corresponding to each sliding moment, determining a window sequence corresponding to each sliding moment, and calculating an average surface electromyography signal amplitude of the window sequence corresponding to each sliding moment, where there is one sliding interval between each sliding moment, the average surface electromyography signal amplitude of the window sequence is an average absolute value of amplitudes of surface electromyography signals within the window sequence, and the window sequence corresponding to the sliding moment includes a total of N consecutive windows: a window corresponding to the sliding moment and N-1 windows corresponding to N-1 sliding moments before the sliding moment, where N is a positive integer greater than or equal to 2; and when an average surface electromyography signal amplitude of a window sequence corresponding to a sliding moment T in all sliding moments is not less than a preset amplitude, using a start time of the window sequence corresponding to the sliding moment T as a start time of the valid surface electromyography signal, adding a preset time to the start time of the valid surface electromyography signal to obtain an end time of the valid surface electromyography signal, and capturing and using a surface electromyography signal of the multiple channels that is located between the start time and the end time of the valid surface electromyography signal as the valid surface electromyography signal, where the preset amplitude may be an average absolute value of amplitudes of the signals obtained by overlapping the surface electromyography signals of the multiple channels.

Step 303. Determine a frequency of the valid surface electromyography signal.

The determining a frequency of the valid surface electromyography signal is specifically: calculating a correlation coefficient between the valid surface electromyography signal and each of multiple sine-cosine matrices, where the sine-cosine matrix includes fundamental-frequency and multiplied-frequency sine functions and cosine functions, and all of the sine-cosine matrices have different fundamental frequencies; determining whether a greatest correlation coefficient in the correlation coefficients between the valid surface electromyography signal and the multiple sine-cosine matrices is greater than a preset correlation coefficient; and if the greatest correlation coefficient is greater than the preset correlation coefficient, using a fundamental frequency of a sine-cosine matrix corresponding to the greatest correlation coefficient as the frequency of the valid surface electromyography signal.

For specific implementation manners of steps 301 to 303, reference may be made to related descriptions in Embodiment 1 and Embodiment 2, and details are not described herein.

Step 304. Extract an amplitude feature of the valid surface electromyography signal.

The extracting an amplitude feature of the valid surface electromyography signal is specifically: first, separately performing sliding window processing on surface electromyography signals of each channel of the valid surface electromyography signal to obtain multiple sliding widows, where for example, a sliding window width is 100 ms, and a sliding interval is 100 ms; and then, calculating an average amplitude of each sliding window of the surface electromyography signals of each channel of the valid surface electromyography signal, where the average amplitude of each sliding window is an average absolute value of amplitudes of surface electromyography signals within each sliding window, and using the average amplitude of each sliding window of the valid surface electromyography signal as the amplitude feature of the valid surface electromyography signal.

It should be noted that in this embodiment, there is no execution sequence between step 303 and step 304, and step 304 may be performed before step 303.

Step 305. Determine, according to the amplitude feature of the valid surface electromyography signal and the frequency of the valid surface electromyography signal, a body action corresponding to the surface electromyography signals of the multiple channels.

Specifically, multiple alternative body actions corresponding to the surface electromyography signals of the multiple channels are first determined according to the frequency of the valid surface electromyography signal. In this embodiment, each frequency may be corresponding to multiple body actions, for example, a 1 HZ frequency may be separately corresponding to the following three body actions: "clenching a fist", "OK gesture", and "bending an elbow". In this case, when it is determined that a frequency of a surface electromyography signal is 1 HZ, the surface electromyography signal is corresponding to the foregoing three alternative body actions. Then, matching is performed between the amplitude feature of the valid surface electromyography signal and an amplitude feature of the multiple alternative body actions that is obtained by means of pre-training, to obtain a body action matched with the amplitude feature of the valid surface electromyography signal, and the body action matched with the amplitude feature of the valid surface electromyography signal is used as the body action corresponding to the surface electromyography signals of the multiple channels. A body action corresponding to a surface electromyography signal may be recognized by using the amplitude feature of the alternative body actions as a template and using a linear discriminant analysis (Linear Discriminant Analysis, LDA for short) classifier.

According to the method in this embodiment, a frequency and an amplitude feature of a surface electromyography signal are obtained, and a body action corresponding to the surface electromyography signal is recognized according to the frequency and amplitude feature of the surface electromyography signal, which not only can improve accuracy of action recognition based on a surface electromyography signal, but also can increase recognition types of surface electromyography signals. Because rhythmic actions, of a same body action, conducted at different frequencies may be regarded as different body actions, in this embodiment, a quantity of recognition types is significantly increased.

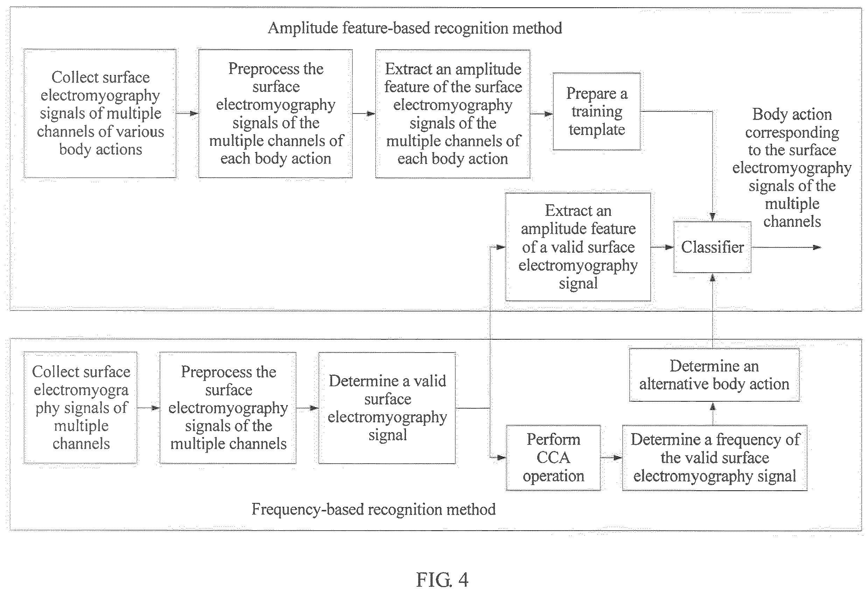

When a body action corresponding to a surface electromyography signal is determined with reference to a frequency and amplitude feature of the surface electromyography signal, the surface electromyography signal needs to be pre-trained to obtain amplitude features of various types of body actions. As shown in FIG. 4, FIG. 4 is an overall block diagram of a recognition method based on a surface electromyography signal according to Embodiment 4 of the present invention. According to the recognition method in this embodiment, a recognition process based on a surface electromyography signal is classified into two parts: a recognition method based on an amplitude feature and a recognition method based on a frequency.

In the recognition method based on an amplitude feature, a surface electromyography signal first needs to be trained, to obtain a training template. A process of training a surface electromyography signal specifically includes the following steps.

Step 1: Collect surface electromyography signals of multiple channels of various body actions.

A user is required to repeat different body actions (for example, "clenching a fist" and "OK gesture") for multiple times. Surface electromyography signals of a main muscle group related to a body action are collected by using a collection apparatus, the collected surface electromyography signals include multiple channels, and a time of each collection is recorded.

Step 2: Preprocess the surface electromyography signals of the multiple channels of each body action.

For example, a 50 HZ power frequency interference trap is performed on the collected surface electromyography signals of the multiple channels, and an FIR filter is used to perform high-pass filtering, to obtain preprocessed surface electromyography signals.

Step 3: Extract amplitude features of the surface electromyography signal of the multiple channels of each body action.

For a specific extraction method, reference may be made to a description of step 304 in Embodiment 3, and details are not described herein.

For example, the user repeats an action of "clenching a fist" for 30 times, start times of all collections are [t1, t2, ..., t30], and collected surface electromyography signals include signals of 8 channels. When amplitude features of the surface electromyography signals are collected, the signals of each channel in the preprocessed surface electromyography signals are slid at a 100 ms sliding window interval successively in a total of 30 time segments: (t1, t1+300 ms), (t2, t2+300 ms), ..., and (t3, t3+300 ms), where a sliding window width is also 100 ms. An average absolute value M(n) of signals of each window in the 30 time segments is calculated, and M(n) is used as the amplitude feature of the action of clenching a fist. Amplitude features of all body actions may be obtained according to the same method.

Step 4: Prepare a training template, that is, establish a correspondence between each body action and an amplitude feature, and then, send the prepared training template to a classifier, so that the classifier recognizes a body action according to the training template.

The foregoing four steps are a training phase, and in a recognition phase, an amplitude feature of a surface electromyography signal also needs to be extracted, and then, the amplitude feature is inputted into a classifier for recognition.

In the frequency-based recognition method, a surface electromyography signal does not need to be trained. In the training phase, at step 1, surface electromyography signals of multiple channels are collected; at step 2, the surface electromyography signals of the multiple channels are preprocessed; at step 3, a frequency of a valid surface electromyography signal is determined; at step 4, CCA operation is performed, that is, a correlation coefficient between the valid surface electromyography signal and each of multiple sine-cosine matrices is calculated to obtain a greatest correlation coefficient of the valid surface electromyography signal; and at step 5, the frequency of the valid surface electromyography signal is determined. For a specific implementation manner of the foregoing step 1 to step 5, reference may be made to descriptions of Embodiment 1 and Embodiment 2, and details are not described herein. After the frequency of the valid surface electromyography signal is determined, an alternative body action is determined according to the frequency of the valid surface electromyography signal.

In this embodiment, after a valid surface electromyography signal is determined, an amplitude feature of the valid surface electromyography signal is extracted, the amplitude feature of the valid surface electromyography signal is inputted into a classifier, the classifier uses an amplitude feature of an alternative body action as a template, and feature matching is performed according to the inputted amplitude feature of the valid surface electromyography signal and the amplitude feature of the alternative body action, to obtain a body action corresponding to the valid surface electromyography signal.

The method in each embodiment of the present invention is applied to a wide range of scenarios: (1) use in an enabling command of a wearable device, for example, to enable an electromyography bracelet by flexing a wrist for multiple times at a specific frequency, thereby avoiding a misoperation; (2) use in a non-stationary state, such as driving or running, for example, for adjusting music volume, switching a song, answering a call, or the like; (3) application to triggering a control instruction due to high recognition accuracy, for example, a handicapped person controls a direction and speed of a wheelchair by using gestures of different rhythms; (4) air mouse, for example, granting finger rotations at different frequencies with corresponding mouse operations; (5) simple game control, for example, two actions: fast and slow actions of a finger can be respectively corresponding to acceleration and brake in a racing game; and (6) use in a rehabilitation process to test physical coordination and a control ability of a user.

Matching is performed between the amplitude feature of the valid surface electromyography signal and the amplitude feature of the multiple alternative body actions that is obtained by means of pre-training, to obtain a body action matched with the amplitude feature of the valid surface electromyography signal, and the body action matched with the amplitude feature of the valid surface electromyography signal is used as a body action corresponding to the surface electromyography signals of the multiple channels.

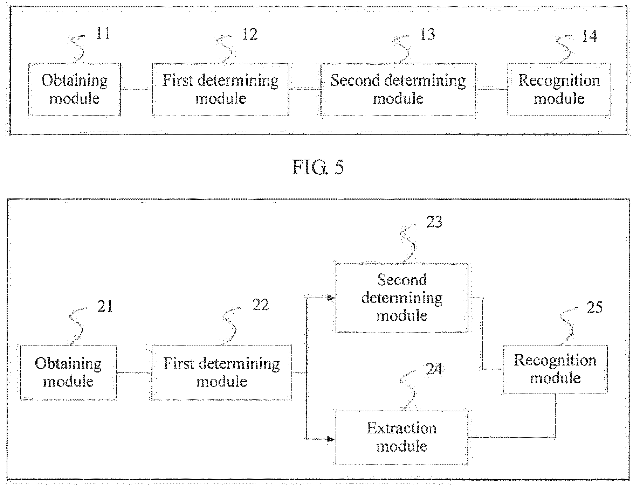

FIG. 5 is a schematic structural diagram of an action recognition device based on a surface electromyography signal according to Embodiment 5 of the present invention. As shown in FIG. 5, the action recognition device based on a surface electromyography signal in this embodiment includes: an obtaining module 11, a first determining module 12, a second determining module 13, and a recognition module 14.

The obtaining module 11 is configured to obtain surface electromyography signals of multiple channels;
the first determining module 12 is configured to determine a valid surface electromyography signal according to the surface electromyography signals of the multiple channels;
the second determining module 13 is configured to determine a frequency of the valid surface electromyography signal; and
the recognition module 14 is configured to determine, according to the frequency of the valid surface electromyography signal, a body action corresponding to the surface electromyography signals of the multiple channels.

Optionally, the first determining module 12 is specifically configured to:

overlap the surface electromyography signals of the multiple channels, and divide the overlapped surface electromyography signals by a quantity of the channels to obtain a single-channel surface electromyography signal;
slide, starting from a start time of the single-channel surface electromyography signal, the single-channel surface electromyography signal at each sliding moment to obtain a window corresponding to each sliding moment, determine, a window sequence corresponding to each sliding moment, and calculate an average surface electromyography signal amplitude of the window sequence corresponding to each sliding moment, where there is one sliding interval between each sliding moment, the average surface electromyography signal amplitude of the window sequence is an average absolute value of amplitudes of surface electromyography signals within the window sequence, and the window sequence corresponding to the sliding moment includes a total of N consecutive windows: a window corresponding to the sliding moment and N-1 windows corresponding to N-1 sliding moments before the sliding moment, where N is a positive integer greater than or equal to 2; and

if an average surface electromyography signal amplitude of a window sequence corresponding to a sliding moment T in all sliding moments is not less than a preset amplitude, use a start time of the window sequence corresponding to the sliding moment T as a start time of the valid surface electromyography signal, add a preset time to the start time of the valid surface electromyography signal to obtain an end time of the valid surface electromyography signal, and capture and use a surface electromyography signal of the multiple channels that is located between the start time and the end time of the valid surface electromyography signal as the valid surface electromyography signal, where the preset amplitude may be an average absolute value of amplitudes of the surface electromyography signals obtained by overlapping the surface electromyography signals of the multiple channels.

Optionally, the second determining module 13 is specifically configured to: first, calculate a correlation coefficient between the valid surface electromyography signal and each of multiple sine-cosine matrices, where the sine-cosine matrix includes fundamental-frequency and multiplied-frequency sine functions and cosine functions, and all of the sine-cosine matrices have different fundamental frequencies; then, determine whether a greatest correlation coefficient in the correlation coefficients between the valid surface electromyography signal and the multiple sine-cosine matrices is greater than a preset correlation coefficient; and if the greatest correlation coefficient is greater than the preset correlation coefficient, use a fundamental frequency of a sine-cosine matrix corresponding to the greatest correlation coefficient as the frequency of the valid surface electromyography signal.

The device in this embodiment of the present invention can be configured to execute the solutions in method Embodiment 1 and Embodiment 2. Specific implementation manners and technical effects are similar, and details are not described herein.

FIG. 6 is a schematic structural diagram of an action recognition device based on a surface electromyography signal according to Embodiment 6 of the present invention. As shown in FIG. 6, the action recognition device based on a surface electromyography signal provided in this embodiment includes: an obtaining module 21, a first determining module 22, a second determining module 23, an extraction module 24, and a recognition module 25.

The obtaining module 21 is configured to obtain surface electromyography signals of multiple channels;

the first determining module 22 is configured to determine a valid surface electromyography signal according to the surface electromyography signals of the multiple channels;

the second determining module 23 is configured to determine a frequency of the valid surface electromyography signal;

the extraction module 24 is configured to extract an amplitude feature of the valid surface electromyography signal; and

the recognition module 25 is configured to determine, according to the amplitude feature of the valid surface electromyography signal and the frequency of the valid surface electromyography signal, a body action corresponding to the surface electromyography signals of the multiple channels.

Optionally, the first determining module 22 is specifically configured to:

overlap the surface electromyography signals of the multiple channels, and divide the overlapped surface electromyography signals by a quantity of the channels to obtain a single-channel surface electromyography signal;

slide, starting from a start time of the single-channel surface electromyography signal, the single-channel surface electromyography signal at each sliding moment to obtain a window corresponding to each sliding moment, determine a window sequence corresponding to each sliding moment, and calculate an average surface electromyography signal amplitude of the window sequence corresponding to each sliding moment, where there is one sliding interval between each sliding moment, the average surface electromyography signal amplitude of the window sequence is an average absolute value of amplitudes of surface electromyography signals within the window sequence, and the window sequence corresponding to the sliding moment includes a total of N consecutive windows: a window corresponding to the sliding moment and N-1 windows corresponding to N-1 sliding moments before the sliding moment, where N is a positive integer greater than or equal to 2; and

when an average surface electromyography signal amplitude of a window sequence corresponding to a sliding moment T in all sliding moments is not less than a preset amplitude, use a start time of the window sequence corresponding to the sliding moment T as a start time of the valid surface electromyography signal, adding a preset time to the start time of the valid surface electromyography signal to obtain an end time of the valid surface electromyography signal, and capture and use a surface electromyography signal of the multiple channels that is located between the start time and the end time of the valid surface electromyography signal as the valid surface electromyography signal, where the preset amplitude may be an average absolute value of amplitudes of the surface electromyography signals obtained by overlapping the surface electromyography signals of the multiple channels.

Optionally, the second determining module 23 is specifically configured to: first, calculate a correlation coefficient between the valid surface electromyography signal and each of multiple sine-cosine matrices, where the sine-cosine matrix includes fundamental-frequency and multiplied-frequency sine functions and cosine functions, and all of the sine-cosine matrices have different fundamental frequencies; then, determine whether a greatest correlation coefficient in the correlation coefficients between the valid surface electromyography signal and the multiple sine-cosine matrices is greater than a preset correlation coefficient; and if the greatest correlation coefficient is greater than the preset correlation coefficient, use a fundamental frequency of a sine-cosine matrix corresponding to the greatest correlation coefficient as the frequency of the valid surface electromyography signal.

Optionally, the extraction module 24 is specifically configured to: separately perform sliding window processing on surface electromyography signals of each channel of the valid surface electromyography signal; and calculate an average amplitude of each sliding window of the surface electromyography signals of each channel of the valid surface electromyography signal, where the average amplitude of each sliding window is an average absolute value of amplitudes of surface electromyography signals within each sliding window, and use the average amplitude of each sliding window of the valid surface electromyography signal as the amplitude feature of the valid surface electromyography signal.

Optionally, the recognition module 25 is specifically configured to: determine, according to the frequency of the valid surface electromyography signal, multiple alternative body actions corresponding to the surface electromyography signals of the multiple channels; and perform matching between the amplitude feature of the valid surface electromyography signal and an amplitude feature of the multiple alternative body actions that is obtained by means of pre-training, to obtain a body action matched with the amplitude feature of the valid surface electromyography signal, and use the body action matched with the amplitude feature of the valid surface electromyography signal as the body action corresponding to the surface electromyography signals of the multiple channels.

The device in this embodiment may be configured to execute the technical solution provided in Embodiment 3. Specific implementation manners and technical effects are similar, and details are not described herein.

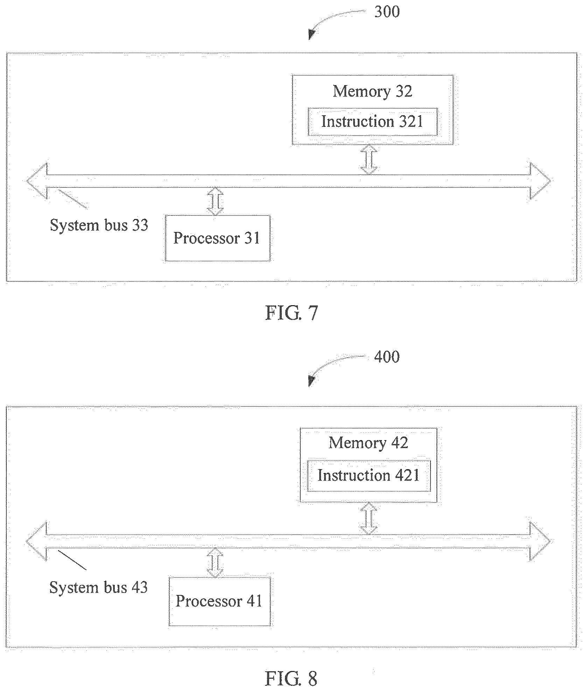

FIG. 7 is a schematic structural diagram of an action recognition device based on a surface electromyography signal according to Embodiment 7 of the present invention. As shown in FIG. 7, an action recognition device 300 based on a surface electromyography signal in this embodiment includes: a processor 31, a memory 32, and a system bus 33. By using the system bus 33, the processor 31 and the memory 32 are connected and implement mutual communication; the memory 32 is configured to store a computer execution instruction 321; the processor 31 is configured to run the computer execution instruction 321, to execute the following method:

obtaining surface electromyography signals of multiple channels;
determining a valid surface electromyography signal according to the surface electromyography signals of the multiple channels;
determining a frequency of the valid surface electromyography signal; and
determining, according to the frequency of the valid surface electromyography signal, a body action corresponding to the surface electromyography signals of the multiple channels.

Optionally, when determining the valid surface electromyography signal according to the surface electromyography signals of the multiple channels, the processor 31 is specifically configured to:

overlap the surface electromyography signals of the multiple channels, and divide the overlapped surface electromyography signals by a quantity of the channels to obtain a single-channel surface electromyography signal;
slide, starting from a start time of the single-channel surface electromyography signal, the single-channel surface electromyography signal at each sliding moment to obtain a window corresponding to each sliding moment, determine a window sequence corresponding to each sliding moment, and calculate an average surface electromyography signal amplitude of the window sequence corresponding to each sliding moment, where there is one sliding interval between each sliding moment, the average surface electromyography signal amplitude of the window sequence is an average absolute value of amplitudes of surface electromyography signals within the window sequence, and the window sequence corresponding to the sliding moment includes a total of N consecutive windows: a window corresponding to the sliding moment and N-1 windows corresponding to N-1 sliding moments before the sliding moment, where N is a positive integer greater than or equal to 2; and
if an average surface electromyography signal amplitude of a window sequence corresponding to a sliding moment T in all sliding moments is not less than a preset amplitude, use a start time of the window sequence corresponding to the sliding moment T as a start time of the valid surface electromyography signal, add a preset time to the start time of the valid surface electromyography signal to obtain an end time of the valid surface electromyography signal, and capture and use a surface electromyography signal of the multiple channels that is located between the start time and the end time of the valid surface electromyography signal as the valid surface electromyography signal, where the preset amplitude may be an average absolute value of amplitudes of the surface electromyography signals obtained by overlapping the surface electromyography signals of the multiple channels.

Optionally, when determining the frequency of the valid surface electromyography signal, the processor 31 is specifically configured to: calculate a correlation coefficient between the valid surface electromyography signal and each of multiple sine-cosine matrices, where the sine-cosine matrix includes fundamental-frequency and multiplied-frequency sine functions and cosine functions, and all of the sine-cosine matrices have different fundamental frequencies; determine whether a greatest correlation coefficient in the correlation coefficients between the valid surface electromyography signal and the multiple sine-cosine matrices is greater than a preset correlation coefficient; and if the greatest correlation coefficient is greater than the preset correlation coefficient, use a fundamental frequency of a sine-cosine matrix corresponding to the greatest correlation coefficient as the frequency of the valid surface electromyography signal.

The device in this embodiment may be configured to execute the technical solutions in Embodiment 1 and Embodiment 2. Specific implementation manners and technical effects are similar, and details are not described herein.

FIG. 8 is a schematic structural diagram of an action recognition device based on a surface electromyography signal according to Embodiment 8 of the present invention. As shown in FIG, 8, an action recognition device 400 based on a surface electromyography signal in this embodiment includes: a processor 41, a memory 42, and a system bus 43. By using the system bus 43, the processor 41 and the memory 42 are connected and implement mutual communication; the memory 42 is configured to store a computer execution instruction 421; the processor 41 is configured to run the computer execution instruction 421, to execute the following method:

obtaining surface electromyography signals of multiple channels;
determining a valid surface electromyography signal according to the surface electromyography signals of the multiple channels;
determining a frequency of the valid surface electromyography signal;
extracting an amplitude feature of the valid surface electromyography signal; and
determining, according to the amplitude feature of the valid surface electromyography signal and the frequency of the valid surface electromyography signal, a body action corresponding to the surface electromyography signals of the multiple channels.

Optionally, when determining the valid surface electromyography signal according to the surface electromyography signals of the multiple channels, the processor 41 is specifically configured to:

overlap the surface electromyography signals of the multiple channels, and divide the overlapped surface electromyography signals by a quantity of the channels to obtain a single-channel surface electromyography signal; slide, starting from a start time of the single-channel surface electromyography signal, the single-channel surface electromyography signal at each sliding moment to obtain a window corresponding to each sliding moment, determine a window sequence corresponding to each sliding moment, and calculate an average surface electromyography signal amplitude of the window sequence corresponding to each sliding moment, where there is one sliding interval between each sliding moment, the average surface electromyography signal amplitude of the window sequence is an average absolute value of amplitudes of surface electromyography signals within the window sequence, and the window sequence corresponding to the sliding moment includes a total of N consecutive windows: a window corresponding to the sliding moment and N-1 windows corresponding to N-1 sliding moments before the sliding moment, where N is a positive integer greater than or equal to 2; and
when an average surface electromyography signal amplitude of a window sequence corresponding to a sliding moment T in all sliding moments is not less than a preset amplitude, use a start time of the window sequence corresponding to the sliding moment T as a start time of the valid surface electromyography signal, adding a preset time to the start time of the valid surface electromyography signal to obtain an end time of the valid surface electromyography signal, and capture and use a surface electromyography signal of the multiple channels that is located between the start time and the end time of the valid surface electromyography signal as the valid surface electromyography signal, where the preset amplitude may be an average absolute value of amplitudes of the surface electromyography signals obtained by overlapping the surface electromyography signals of the multiple channels.

Optionally, when determining the frequency of the valid surface electromyography signal, the processor 41 is specifically configured to: calculate a correlation coefficient between the valid surface electromyography signal and each of multiple sine-cosine matrices, where the sine-cosine matrix includes fundamental-frequency and multiplied-frequency sine functions and cosine functions, and all of the sine-cosine matrices have different fundamental frequencies; determine whether a greatest correlation coefficient in the correlation coefficients between the valid surface electromyography signal and the multiple sine-cosine matrices is greater than a preset correlation coefficient; and if the greatest correlation coefficient is greater than the preset correlation coefficient, use a fundamental frequency of a sine-cosine matrix corresponding to the greatest correlation coefficient as the frequency of the valid surface electromyography signal.

Optionally, when extracting the amplitude feature of the valid surface electromyography signal, the processor 41 is specifically configured to: separately perform sliding window processing on surface electromyography signals of each channel of the valid surface electromyography signal; and calculate an average amplitude of each sliding window of the surface electromyography signals of each channel of the valid surface electromyography signal, where the average amplitude of each sliding window is an average absolute value of amplitudes of surface electromyography signals within each sliding window, and use the average amplitude of each sliding window of the valid surface electromyography signal as the amplitude feature of the valid surface electromyography signal.

Optionally, when determining, according to the amplitude feature of the valid surface electromyography signal and the frequency of the valid surface electromyography signal, the body action corresponding to the surface electromyography signals of the multiple channels, the processor 41 is specifically configured to: determine, according to the frequency of the valid surface electromyography signal, multiple alternative body actions corresponding to the surface electromyography signals of the multiple channels; and perform matching between the amplitude feature of the valid surface electromyography signal and an amplitude feature of the multiple alternative body actions that is obtained by means of pre-training, to obtain a body action matched with the amplitude feature of the valid surface electromyography signal, and use the body action matched with the amplitude feature of the valid surface electromyography signal as the body action corresponding to the surface electromyography signals of the multiple channels.

The device in this embodiment may be configured to execute the technical solution in Embodiment 3. Specific implementation manners and technical effects are similar, and details are not described herein.

Persons of ordinary skill in the art may understand that all or some of the steps of the method embodiments may be implemented by a program instructing relevant hardware. The program may be stored in a computer-readable storage medium. When the program runs, the steps of the method embodiments are performed. The foregoing storage medium includes: any medium that can store program code, such as a ROM, a RAM, a magnetic disk, or an optical disc.

Finally, it should be noted that the foregoing embodiments are merely intended for describing the technical solutions of the present invention, but not for limiting the present invention. Although the present invention is described in detail with reference to the foregoing embodiments, persons of ordinary skill in the art should understand that they may still make modifications to the technical solutions described in the foregoing embodiments or make equivalent replacements to some or all technical features thereof, without departing from the scope of the technical solutions of the embodiments of the present invention.

**Claims**

1. An action recognition method based on a surface electromyography signal, comprising:

   obtaining surface electromyography signals of multiple channels;
   determining a valid surface electromyography signal according to the surface electromyography signals of the multiple channels;
   determining a frequency of the valid surface electromyography signal; and
   determining, according to the frequency of the valid surface electromyography signal, a body action corresponding to the surface electromyography signals of the multiple channels.

2. The method according to claim 1, wherein the determining a valid surface electromyography signal according to the surface electromyography signals of the multiple channels comprises:

   overlapping the surface electromyography signals of the multiple channels, and dividing the overlapped surface electromyography signals by a quantity of the channels to obtain a single-channel surface electromyography signal;
   sliding, starting from a start time of the single-channel surface electromyography signal, the single-channel surface electromyography signal at each sliding moment to obtain a window corresponding to each sliding moment, determining a window sequence corresponding to each sliding moment, and calculating an average surface electromyography signal amplitude of the window sequence corresponding to each sliding moment, wherein there is one sliding interval between each sliding moment, the average surface electromyography signal amplitude of the window sequence is an average absolute value of amplitudes of surface electromyography signals within the window sequence, and the window sequence corresponding to the sliding moment comprises a total of N consecutive windows: a window corresponding to the sliding moment and N-1 windows corresponding to N-1 sliding moments before the sliding moment, wherein N is a positive integer greater than or equal to 2; and if an average surface electromyography signal amplitude of a window sequence corresponding to a sliding moment T in all sliding moments is not less than a preset amplitude, using a start time of the window sequence corresponding to the sliding moment T as a start time of the valid surface electromyography signal, adding a preset time to the start time of the valid surface electromyography signal to obtain an end time of the valid surface electromyography signal, and capturing and using a surface electromyography signal of the multiple channels that is located between the start time and the end time of the valid surface electromyography signal as the valid surface electromyography signal.

3. The method according to claim 2, wherein the preset amplitude is an average absolute value of amplitudes of the surface electromyography signals obtained by overlapping the surface electromyography signals of the multiple channels.

4. The method according to any one of claims 1 to 3, wherein the determining a frequency of the valid surface electromyography signal comprises:

Calculating a correlation coefficient between the valid surface electromyography signal and each of multiple sine-cosine matrices, wherein the sine-cosine matrix comprises fundamental-frequency and multiplied-frequency sine functions and cosine functions, and all of the sine-cosine matrices have different fundamental frequencies; determining whether a greatest correlation coefficient in the correlation coefficients between the valid surface electromyography signal and the multiple sine-cosine matrices is greater than a preset correlation coefficient; and if the greatest correlation coefficient is greater than the preset correlation coefficient, using a fundamental frequency of a sine-cosine matrix corresponding to the greatest correlation coefficient as the frequency of the valid surface electromyography signal.

5. An action recognition method based on a surface electromyography signal, comprising:

obtaining surface electromyography signals of multiple channels;
determining a valid surface electromyography signal according to the surface electromyography signals of the multiple channels;
determining a frequency of the valid surface electromyography signal;
extracting an amplitude feature of the valid surface electromyography signal; and
determining, according to the amplitude feature of the valid surface electromyography signal and the frequency of the valid surface electromyography signal, a body action corresponding to the surface electromyography signals of the multiple channels.

6. The method according to claim 5, wherein the determining a valid surface electromyography signal according to the surface electromyography signals of the multiple channels comprises:

overlapping the surface electromyography signals of the multiple channels, and dividing the overlapped surface electromyography signals by a quantity of the channels to obtain a single-channel surface electromyography signal;
sliding, starting from a start time of the single-channel surface electromyography signal, the single-channel surface electromyography signal at each sliding moment to obtain a window corresponding to each sliding moment, determining a window sequence corresponding to each sliding moment, and calculating an average surface electromyography signal amplitude of the window sequence corresponding to each sliding moment, wherein there is one sliding interval between each sliding moment, the average surface electromyography signal amplitude of the window sequence is an average absolute value of amplitudes of surface electromyography signals within the window sequence, and the window sequence corresponding to the sliding moment comprises a total of N consecutive windows: a window corresponding to the sliding moment and N-1 windows corresponding to N-1 sliding moments before the sliding moment, wherein N is a positive integer greater than or equal to 2; and when an average surface electromyography signal amplitude of a window sequence corresponding to a sliding moment T in all sliding moments is not less than a preset amplitude, using a start time of the window sequence corresponding to the sliding moment T as a start time of the valid surface electromyography signal, adding a preset time to the start time of the valid surface electromyography signal to obtain an end time of the valid surface electromyography signal, and capturing and using a surface electromyography signal of the multiple channel that is located between the start time and the end time of the valid surface electromyography signal as the valid surface electromyography signal.

7. The method according to claim 6, wherein the preset amplitude is an average absolute value of amplitudes of the surface electromyography signals obtained by overlapping the surface electromyography signals of the multiple channels.

8. The method according to any one of claims 5 to 7, wherein the determining a frequency of the valid surface electromyography signal comprises:

calculating a correlation coefficient between the valid surface electromyography signal and each of multiple sine-cosine matrices, wherein the sine-cosine matrix comprises fundamental-frequency and multiplied-frequency sine functions and cosine functions, and all of the sine-cosine matrices have different fundamental frequencies; determining whether a greatest correlation coefficient in the correlation coefficients between the valid surface electromyography signal and the multiple sine-cosine matrices is greater than a preset correlation coefficient; and if the greatest correlation coefficient is greater than the preset correlation coefficient, using a fundamental frequency of a sine-cosine matrix corresponding to the greatest correlation coefficient as the frequency of the valid surface electromyography signal.

9. The method according to claim 8, wherein the extracting an amplitude feature of the valid surface electromyography signal comprises:

separately performing sliding window processing on surface electromyography signals of each channel of the valid surface electromyography signal; and
calculating an average amplitude of each sliding window of the surface electromyography signals of each channel of the valid surface electromyography signal, wherein the average amplitude of each sliding window is an average absolute value of amplitudes of surface electromyography signals within each sliding window, and using the average amplitude of each sliding window of the valid surface electromyography signal as the amplitude feature of the valid surface electromyography signal.

10. The method according to any one of claims 5 to 7, wherein the determining, according to the amplitude feature of the valid surface electromyography signal and the frequency of the valid surface electromyography signal, a body action corresponding to the surface electromyography signals of the multiple channels comprises:

determining, according to the frequency of the valid surface electromyography signal, multiple alternative body actions corresponding to the surface electromyography signals of the multiple channels; and
performing matching between the amplitude feature of the valid surface electromyography signal and an amplitude feature of the multiple alternative body actions that is obtained by means of pre-training, to obtain a body action matched with the amplitude feature of the valid surface electromyography signal, and using the body action matched with the amplitude feature of the valid surface electromyography signal as the body action corresponding to the surface electromyography signals of the multiple channels.

11. An action recognition device based on a surface electromyography signal, comprising:

an obtaining module, configured to obtain surface electromyography signals of multiple channels;
a first determining module, configured to determine a valid surface electromyography signal according to the surface electromyography signals of the multiple channels;
a second determining module, configured to determine a frequency of the valid surface electromyography signal; and
a recognition module, configured to determine, according to the frequency of the valid surface electromyography signal, a body action corresponding to the surface electromyography signals of the multiple channels.

12. The device according to claim 11, wherein the first determining module is specifically configured to:

overlap the surface electromyography signals of the multiple channels, and divide the overlapped surface electromyography signals by a quantity of the channels to obtain a single-channel surface electromyography signal;
sliding, starting from a start time of the single-channel surface electromyography signal, the single-channel surface electromyography signal at each sliding moment to obtain a window corresponding to each sliding moment, determine a window sequence corresponding to each sliding moment, and calculate an average surface electromyography signal amplitude of the window sequence corresponding to each sliding moment, wherein there is one sliding interval between each sliding moment, the average surface electromyography signal amplitude of the window sequence is an average absolute value of amplitudes of surface electromyography signals within the window sequence, and the window sequence corresponding to the sliding moment comprises a total of N consecutive windows: a window corresponding to the sliding moment and N-1 windows corresponding to N-1 sliding moments before the sliding moment, wherein N is a positive integer greater than or equal to 2; and if an average surface electromyography signal amplitude of a window sequence corresponding to a sliding moment T in all sliding moments is not less than a preset amplitude, use a start time of the window sequence corresponding to the sliding moment T as a start time of the valid surface electromyography signal, add a preset time to the start time of the valid surface electromyography signal to obtain an end time of the valid surface electromyography signal, and capture and use a surface electromyography signal of the multiple channels that is located between the start time and the end time of the valid surface electromyography signal as the valid surface electromyography signal.

13. The device according to claim 12, wherein the preset amplitude is an average absolute value of amplitudes of the surface electromyography signals obtained by overlapping the surface electromyography signals of the multiple channels.

14. The device according to any one of claims 11 to 13, wherein the second determining module is specifically configured to:

calculate a correlation coefficient between the valid surface electromyography signal and each of multiple sine-cosine matrices, wherein the sine-cosine matrix comprises fundamental-frequency and multiplied-frequency sine functions and cosine functions, and all of the sine-cosine matrices have different fundamental frequencies; determine whether a greatest correlation coefficient in the correlation coefficients between the valid surface electromyography signal and the multiple sine-cosine matrices is greater than a preset correlation coefficient; and if the greatest correlation coefficient is greater than the preset correlation coefficient, use a fundamental frequency of a sine-cosine matrix corresponding to the greatest correlation coefficient as the frequency of the valid surface electromyography signal.

15. An action recognition device based on a surface electromyography signal, comprising:

an obtaining module, configured to obtain surface electromyography signals of multiple channels; a first determining module, configured to determine a valid surface electromyography signal according to the surface electromyography signals of the multiple channels; a second determining module, configured to determine a frequency of the valid surface electromyography signal; an extraction module, configured to extract an amplitude feature of the valid surface electromyography signal; and a recognition module, configured to determine, according to the amplitude feature of the valid surface electromyography signal and the frequency of the valid surface electromyography signal, a body action corresponding to the surface electromyography signals of the multiple channels.

16. The device according to claim 15, wherein the first determining module is specifically configured to:

overlap the surface electromyography signals of the multiple channels, and divide the overlapped surface electromyography signals by a quantity of the channels to obtain a single-channel surface electromyography signal; slide, starting from a start time of the single-channel surface electromyography signal, the single-channel surface electromyography signal at each sliding moment to obtain a window corresponding to each sliding moment, determine a window sequence corresponding to each sliding moment, and calculate an average surface electromyography signal amplitude of the window sequence corresponding to each sliding moment, wherein there is one sliding interval between each sliding moment, the average surface electromyography signal amplitude of the window sequence is an average absolute value of amplitudes of surface electromyography signals within the window sequence, and the window sequence corresponding to the sliding moment comprises a total of N consecutive windows: a window corresponding to the sliding moment and N-1 windows corresponding to N-1 sliding moments before the sliding moment, wherein N is a positive integer greater than or equal to 2; and when an average surface electromyography signal amplitude of a window sequence corresponding to a sliding moment T in all sliding moments is not less than a preset amplitude, use a start time of the window sequence corresponding to the sliding moment T as a start time of the valid surface electromyography signal, adding a preset time to the start time of the valid surface electromyography signal to obtain an end time of the valid surface electromyography signal, and capture and use a surface electromyography signal of the multiple channels that is located between the start time and the end time of the valid surface electromyography signal as the valid surface electromyography signal.

17. The device according to claim 16, wherein the preset amplitude is an average absolute value of amplitudes of the surface electromyography signals obtained by overlapping the surface electromyography signals of the multiple channels.

18. The device according to any one of claims 15 to 17, wherein the second determining module is specifically configured to:

calculate a correlation coefficient between the valid surface electromyography signal and each of multiple sine-cosine matrices, wherein the sine-cosine matrix comprises fundamental-frequency and multiplied-frequency sine functions and cosine functions, and all of the sine-cosine matrices have different fundamental frequencies; determine whether a greatest correlation coefficient in the correlation coefficients between the valid surface electromyography signal and the multiple sine-cosine matrices is greater than a preset correlation coefficient; and if the greatest correlation coefficient is greater than the preset correlation coefficient, use a fundamental frequency of a sine-cosine matrix corresponding to the greatest correlation coefficient as the frequency of the valid surface electromyography signal.

19. The device according to claim 18, wherein the extraction module is specifically configured to:

separately perform sliding window processing on surface electromyography signals of each channel of the valid surface electromyography signal; and
calculate an average amplitude of each sliding window of the surface electromyography signals of each channel of the valid surface electromyography signal, wherein the average amplitude of each sliding window is an average absolute value of amplitudes of surface electromyography signals within each sliding window, and use the average amplitude of each sliding window of the valid surface electromyography signal as the amplitude feature of the valid surface electromyography signal.

20. The device according to any one of claims 15 to 17, wherein the recognition module is specifically configured to:

determine, according to the frequency of the valid surface electromyography signal, multiple alternative body actions corresponding to the surface electromyography signals of the multiple channels; and
perform matching between the amplitude feature of the valid surface electromyography signal and an amplitude feature of the multiple alternative body actions that is obtained by means of pre-training, to obtain a body action matched with the amplitude feature of the valid surface electromyography signal, and use the body action matched with the amplitude feature of the valid surface electromyography signal as the body action corresponding to the surface electromyography signals of the multiple channels.

21. An action recognition device based on a surface electromyography signal, comprising:

a processor, a memory, and a system bus, wherein by using the system bus, the processor and the memory are connected and implement mutual communication;
the memory is configured to store a computer execution instruction; and
the processor is configured to run the computer execution instruction and enable the action recognition device to execute the method according to any one of claims 1 to 4.

22. An action recognition device based on a surface electromyography signal, comprising:

a processor, a memory, and a system bus, wherein by using the system bus, the processor and the memory are connected and implement mutual communication;
the memory is configured to store a computer execution instruction; and
the processor is configured to run the computer execution instruction and enable the action recognition device to execute the method according to any one of claims 5 to 10.

| Obtain surface electromyography signals of multiple channels | 101 |

| Determine a valid surface electromyography signal according to the surface electromyography signals of the multiple channels | 102 |

| Determine a frequency of the valid surface electromyography signal | 103 |

| Determine, according to the frequency of the valid surface electromyography signal, a body action corresponding to the surface electromyography signals of the multiple channels | 104 |

FIG. 1

| Calculate a correlation coefficient between the valid surface electromyography signal and each of multiple sine-cosine matrices, where the sine-cosine matrix includes fundamental-frequency and multiplied-frequency sine functions and cosine functions, and all of the sine-cosine matrices have different fundamental frequencies | 201 |

| Determine whether a greatest correlation coefficient in the correlation coefficients between the valid surface electromyography signal and the multiple sine-cosine matrices is greater than a preset correlation coefficient | 202 |

| If the greatest correlation coefficient is greater than the preset correlation coefficient, use a fundamental frequency of a sine-cosine matrix corresponding to the greatest correlation coefficient as the frequency of the valid surface electromyography signal | 203 |

FIG. 2

Obtain surface electromyography signals of multiple channels — 301

Determine a valid surface electromyography signal according to the surface electromyography signals of the multiple channels — 302

Determine a frequency of the valid surface electromyography signal — 303

Extract an amplitude feature of the valid surface electromyography signal — 304

Determine, according to the amplitude feature of the valid surface electromyography signal and the frequency of the valid surface electromyography signal, a body action corresponding to the surface electromyography signals of the multiple channels — 305

FIG. 3

**Amplitude feature-based recognition method**

```
┌─────────────┐   ┌──────────────┐   ┌──────────────┐   ┌──────────┐
│Collect surface│→ │ Preprocess the│→ │Extract an    │→ │Prepare a │
│electromyography│  │   surface    │   │amplitude     │   │training  │
│signals of     │  │electromyography│ │feature of the│   │template  │
│multiple       │  │signals of the │  │surface       │   │          │
│channels of    │  │multiple       │  │electromyography│ └──────────┘
│various        │  │channels of    │  │signals of the │
│body actions   │  │each body action│ │multiple channels│
└─────────────┘   └──────────────┘   │of each body action│
                                      └──────────────┘
```

Body action corresponding to the surface electromyography signals of the multiple channels

Extract an amplitude feature of a valid surface electromyography signal

Classifier

Determine an alternative body action

**Frequency-based recognition method**

```
┌─────────────┐   ┌──────────────┐   ┌──────────────┐
│Collect surface│→ │ Preprocess the│→ │Determine a valid│
│electromyogra │  │   surface    │   │surface       │
│phy signals of │  │electromyography│ │electromyography│
│multiple       │  │signals of the │  │signal        │
│channels       │  │multiple channels│ │              │
└─────────────┘   └──────────────┘   └──────────────┘
```

Perform CCA operation

Determine a frequency of the valid surface electromyography signal

FIG. 4

```
   ┌─ 11            ┌─ 12                ┌─ 13                ┌─ 14
   │                │                    │                    │
 ┌─────────┐    ┌─────────────┐    ┌──────────────────┐   ┌─────────────┐
 │Obtaining│    │First determining│  │Second determining│   │Recognition  │
 │module   │────│module       │────│module            │───│module       │
 └─────────┘    └─────────────┘    └──────────────────┘   └─────────────┘
```

FIG. 5

```
                                        ┌─ 23
                                        │
                                 ┌──────────────┐
                                 │   Second     │
   ┌─ 21          ┌─ 22          │ determining  │        ┌─ 25
   │              │              │   module     │        │
 ┌──────────┐  ┌──────────────┐  └──────────────┘   ┌─────────────┐
 │Obtaining │  │First determining│                   │Recognition  │
 │module    │──│module        │──┐         ┌─ 24      │module       │
 └──────────┘  └──────────────┘  │         │         └─────────────┘
                                 │  ┌──────────────┐
                                 └─▶│ Extraction   │
                                    │   module     │
                                    └──────────────┘
```

300

Memory 32

Instruction 321

System bus 33

Processor 31

FIG. 7

400

Memory 42

Instruction 421

System bus 43

Processor 41

FIG. 8

# INTERNATIONAL SEARCH REPORT

| International application No. |
| --- |
| **PCT/CN2015/073369** |

**A. CLASSIFICATION OF SUBJECT MATTER**

A61B 5/0488 (2006.01) i

According to International Patent Classification (IPC) or to both national classification and IPC

**B. FIELDS SEARCHED**

Minimum documentation searched (classification system followed by classification symbols)

A61B 5

Documentation searched other than minimum documentation to the extent that such documents are included in the fields searched

Electronic data base consulted during the international search (name of data base and, where practicable, search terms used)

CNABS; CNTXT; DWPI; SIPOABS; USTXT; EPTXT; WOTXT: electromyography, EMG, action, movement, gesture, recognit+, identif+, channel?, frequency, magnitude, window, slid+, mov+, sequence

**C. DOCUMENTS CONSIDERED TO BE RELEVANT**

| Category* | Citation of document, with indication, where appropriate, of the relevant passages | Relevant to claim No. |
| --- | --- | --- |
| A | CN 101995947 A (THE CHINESE UNIVERSITY OF HONG KONG), 30 March 2011 (30.03.2011), description, paragraphs [0020]-[0040] | 1-22 |
| A | CN 103495260 A (SOUTHEAST UNIVERSITY; NANJING SENSECHIP MEDICAL DEVICES CO., LTD.), 08 January 2014 (08.01.2014), the whole document | 1-22 |
| A | CN 103654774 A (BEIJING MEDYNAMICS TECHNOLOGIES CO., LTD.), 26 March 2014 (26.03.2014), the whole document | 1-22 |
| A | US 2007276280 A1 (URBAN, B. et al.), 29 November 2007 (29.11.2007), the whole document | 1-22 |

☐ Further documents are listed in the continuation of Box C.     ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 18 May 2015 (18.05.2015) | **25 May 2015 (25.05.2015)** |

| Name and mailing address of the ISA/CN: State Intellectual Property Office of the P. R. China No. 6, Xitucheng Road, Jimenqiao Haidian District, Beijing 100088, China Facsimile No.: (86-10) 62019451 | Authorized officer **ZHU, Yingying** Telephone No.: (86-10) **62089620** |

Form PCT/ISA/210 (second sheet) (July 2009)

## INTERNATIONAL SEARCH REPORT
Information on patent family members

| International application No. |
| --- |
| **PCT/CN2015/073369** |

| Patent Documents referred in the Report | Publication Date | Patent Family | Publication Date |
| --- | --- | --- | --- |
| CN 101995947 A | 30 March 2011 | CN 101995947 B | 04 July 2012 |
| | | HK 1151598 A1 | 30 November 2012 |
| CN 103495260 A | 08 January 2014 | WO 2014194609 A1 | 11 December 2014 |
| CN 103654774 A | 26 March 2014 | None | |
| US 2007276280 A1 | 29 November 2007 | JP 5361129 B2 | 04 December 2013 |
| | | EP 1686893 B1 | 16 April 2014 |
| | | JP 2007511321 A | 10 May 2007 |
| | | SE 0303061 D0 | 19 November 2003 |
| | | US 7689275 B2 | 30 March 2010 |
| | | EP 1686893 B8 | 16 July 2014 |
| | | WO 2005048839 A1 | 02 June 2005 |
| | | EP 1686893 A1 | 09 August 2006 |

Form PCT/ISA/210 (patent family annex) (July 2009)